# EUROPÄISCHE PATENTANMELDUNG

(11) **EP 3 269 812 A1**
(43) Veröffentlichungstag der Anmeldung: **17.01.2018**
(21) Anmeldenummer: 16179234.6
(22) Anmeldetag: 13.07.2016
(51) Int. Cl.: C12N 15/10

(54) **ZELLFREIE ULTRA-HOCHDURCHSATZ-DURCHMUSTERUNGSSYSTEME FÜR DAS PROTEIN-ENGINEERING**

(71) Anmelder: Rheinisch-Westfälische Technische Hochschule Aachen (RWTH), 52062 Aachen (DE)
(72) Erfinder: Schwaneberg, Ulrich, 52074 Aachen (DE); Vojcic, Ljubica, 52074 Aachen (DE); Körfer, Georgette, 52074 Aachen (DE); Pitzler, Christian, 52074 Aachen (DE); Mertens, Alan, 52074 Aachen (DE); Martinez-Moya, Ronny, 52074 Aachen (DE)
(74) Vertreter: Eisenführ Speiser

(57) **Zusammenfassung**

Die Erfindung betrifft ein Verfahren zur Herstellung einer Vielzahl von zellfreien Kompartimenten für die Durchmusterung von (i) Enzymvarianten und/oder (ii) Varianten fluoreszierender Proteine umfassend die Schritte:
a) Bereitstellen einer Vielzahl von Genvarianten,
b) Bereitstellen einer Vielzahl eines *in vitro* Expressionssystems,
c) Bereitstellen, im Falle von (i), einer Vielzahl mindestens eines Substrates für das Enzym, dessen Varianten durchgemustert werden sollen,
d) Bereitstellen zur Bildung von Polymervesikeln geeigneter Bausteine und
e) Bildung von einer Vielzahl von Polymervesikeln aus den Bausteinen als zellfreie Kompartimente unter Einschluss von wenigstens einer Genvariante, bevorzugt unter Einschluss von genau einer Genvariante, des *in vitro* Expressionssystems und im Falle von (i), des Substrates, wobei die Polymervesikel Einfachkompartimente sind.

SEQUENCE LISTING

<110>  Rheinisch-Westfälische Technische Hochschule Aachen (RWTH)

<120>  Zellfreie Ultra-Hochdurchsatz-Durchmusterungssysteme für das
       Protein-Engineering

<130>  RA 1697-01EP

<160>  4

<170>  PatentIn version 3.5

<210>  1
<211>  31
<212>  DNA
<213>  Artificial Sequence

<220>
<223>  Primer


<220>
<221>  Phosphothioat-Modifikation_des_DNA-Rückgrats
<222>  (1)..(12)

<400>  1
atgagcgaag aatggcagac ctatgaaaaa g                                    31


<210>  2
<211>  33
<212>  DNA
<213>  Artificial Sequence

<220>
<223>  Primer


<220>
<221>  Phosphothioat-Modifikation_des_DNA-Rückgrats
<222>  (1)..(12)

<400>  2
ttattattcg gccacggtat tcaggctata aac                                  33


<210>  3
<211>  28
<212>  DNA
<213>  Artificial Sequence

<220>
<223>  Primer


<220>
<221>  Phosphothioat-Modifikation_des_DNA-Rückgrats
<222>  (1)..(12)

<400>  3
gccgaataat aagaattcga gctcgtcg                                        28

<210>  4
<211>  22
<212>  DNA
<213>  Artificial Sequence

<220>
<223>  Primer



<220>
<221>  Phosphothioat-Modifikation_des_DNA-Rückgrats
<222>  (1)..(12)

<400>  4
ttcttcgctc atgctagcac cc                                                22

## Beschreibung

Die Erfindung betrifft ein Verfahren zur Herstellung einer Vielzahl von zellfreien Kompartimenten für die Durchmusterung von Enzymvarianten oder Varianten fluoreszierender Proteine, ein Verfahren für die Durchmusterung von Enzymvarianten oder von Proteinvarianten in zellfreien Kompartimenten, die Verwendung von Polymervesikeln zur Herstellung einer Vielzahl von zellfreien Kompartimenten für die genannte Durchmusterung, sowie auch eine derartige Vielzahl von zellfreien Kompartimenten selbst und eine diesbezügliche Fraktion von Polymervesikeln umfassend mindestens ein Gen für ein durchzumusterndes Enzym oder Protein.

Nachfolgend wird der Stand der Technik zu Kompartiment-basierten, zellfreien Ultra-Hochdurchsatz-Durchmusterungssystemen für das Protein-Engineering sowie zu in diesem Zusammenhang verwendeten Techniken wie insbesondere *in vitro*-Expression, Kompartimentalisierung, Hochdurchsatz-Durchmusterung, Gelenkte Evolution, Metagenomscreening und Durchflusszytometrie beschrieben.

Um mit Hilfe der Gelenkten Evolution Enzyme, oder generell Proteine, mit maßgeschneiderten Eigenschaften zu evolvieren, können dank moderner Mutagenese-Methoden in kurzer Zeit mehrere Millionen Enzym-Varianten generiert werden. Aus Zeit- und Kostengründen können bei Verwendung von Standard-Screening-Methoden jedoch nur ein paar Tausend und somit nur Bruchteile der erhaltenen Vielfalt an Enzymen, durchgemustert werden. Dieses und niedrige Mutagenese-Frequenzen, die in klassischen Standard-Gelenkten Evolutionsexperimenten eingestellt werden, führen im Stand der Technik dazu, dass oft fünf bis sechs iterative Gelenkte Evolutionsrunden durchgeführt werden müssen, um signifikant verbesserte Enzym-Varianten aufzufinden (z.B. auf Aktivitätsverbesserungen pro Runde zwischen Faktor 1,5- und 2,5-fach abzielend) (siehe Lit. 1). Dementsprechend kosten- und zeitintensiv ist das Auffinden verbesserter Enzym-Varianten, da letztlich durchschnittlich 2 - 3 Wochen pro Gelenkter Evolutionsrunde in *E. coli* und 3 - 4 Wochen pro Gelenkter Evolutionsrunde in Hefen vergehen. Bei sechs Runden pro Evolutionskampagne werden daher bisher im Stand der Technik 12 - 24 Wochen an reiner Arbeitszeit für die Gelenkte Evolution benötigt.

Darüber hinaus ist die Verbesserung und Nutzung von vielen, z.B. toxischen oder humanen, Proteinen im Stand der Technik kaum möglich, da diese Proteine bei Verwendung typischer Systeme zur heterologen Expression (wie *E. coli*, Hefen, CHO-Zelllinien) nicht in funktionaler Form hergestellt werden können. Letzteres unterbindet somit die Nutzung der Gelenkten Evolutionsmethoden zur Verbesserung von Proteineigenschaften.

Weiterhin wurden im Stand der Technik durch funktions- und sequenzbasierte Ansätze bisher viele neue Enzyme (Proteine) und andere wertvolle Biomoleküle in Metagenomen entdeckt (nicht-kultivierbare Biodiversität). Allerdings ist die Erstellung von Metagenombanken und deren funktionale Durchmusterung insbesondere für eukaryontische Enzyme, z.B. aus Pilzen oder höheren Organismen, bisher nicht oder nur mit geringen Erfolgsaussichten möglich. Zugrundeliegende Probleme können dabei die Extraktion und Qualität der Umwelt-DNA sein. Nach erfolgreicher Erstellung folgen häufig Probleme bezüglich schwacher Enzym- bzw. Protein-Expression, Enzym- bzw. Protein-Faltung und/oder Enzym- bzw. Protein-Sekretion der heterologen Enzyme/Proteine im Wirtsstamm, da insbesondere eukaryontische Enzyme/Proteine glykosyliert werden müssen, um wasserlöslich und/oder aktiv zu sein. Ein weiterer limitierender Faktor ist zudem die Transkription in einem "wenig-kompatiblen" Wirtsstamm. Daher besteht im Stand der Technik ein großer Bedarf und ein hohes kommerzielles Interesse an der Entwicklung und/oder Verbesserung von Methoden für die funktionale Durchmusterung von eukaryontischen Genen, um einen effizienten Zugang zu insbesondere eukaryontischen Genen in nichtkultivierbarer Biodiversität (oftmals Mikrobiome) bereitstellen zu können.

Im Stand der Technik wurde bisher versucht, die vorstehend genannten Probleme wie folgt zu lösen. Zum Durchmustern der generierten Diversität im Hochdurchsatz existieren einige Nachweissysteme, die einen Durchsatz von 10⁷ - 10¹⁰ Varianten erreichen. Diese umfassen Durchflusszytometrie-Systeme, Displaytechnologien (z.B. Phage- und Bacterial-Display) und Mikrofluidikansätze (siehe Lit. 2, 3). Displaytechnologien werden insbesondere zur Verbesserung von Bindungsaffinitäten von Proteinen eingesetzt. Trotz signifikanter Fortschritte sind bisher jedoch nur wenige und nur sehr spezielle Lösungen gefunden worden, um mittels Displaytechnologie neben der Affinität auch Eigenschaften wie die Enzymaktivität zu verbessern. Hochdurchsatz-Durchmusterungssysteme basierend auf Mikrofluidik stellen einen neueren Ansatz zur Hochdurchsatz-Durchmusterung dar. Wenngleich große Chancen erwartet werden, befindet sich die Mikrofluidik-Durchmusterungstechnologie heute aber immer noch in einer eher frühen Entwicklungsphase und wird vorwiegend durch die Hardware-Technologieentwicklung getrieben. Ein aussagekräftiger Potentialvergleich zur gerätetechnisch weitentwickelten und seit Jahrzehnten kommerzialisierten Durchflusszytometrie ist zum jetzigen Zeitpunkt daher noch nicht möglich.

Grundlegende Arbeiten zur Durchflusszytometrie mit Kompartimentalisierung von Zellen in Doppelemulsionen wurden im Stand der Technik von Griffiths und Tawfik gelegt (siehe Lit. 4, 5). Bis dato gibt es zwar eine Reihe von Gelenkten Evolutionsexperimenten, in denen Durchflusszytometrie-Durchmusterungssysteme in Doppelemulsionen eingesetzt wurden, Doppelemulsionen besitzen jedoch den Nachteil, dass sie verhältnismäßig aufwendig in der Herstellung sind und nur eine recht geringe Enkapsulierungseffizienz sowie eine recht geringe Stabilität aufweisen.

Doppelemulsionen wurden in der Vergangenheit mittels Tensiden (waschaktiver Substanzen) aufgebaut und für zellfreie (*in vitro*) Expression innerhalb dieser Doppelemulsionen verwendet. Im Detail umfasst die chemische Zusammensetzung der Doppelemulsion im Stand der Technik leichtes Mineralöl und Tenside: Span 80, Tween 80. Die Emulsionen können durch Extrudieren oder Homogenisieren erzeugt werden. Die Einfachemulsion wird beispielsweise durch Extrusionsverfahren erzeugt, durch Zugabe einer Ölhaltigen Lösung (Span 80, Tween 80 in leichtem Mineralöl gelöst) zum wässrigen zellfreien Reaktionsgemisch und durch dreimaliges Extrudieren der Lösung durch eine 5 µm Whatman Membran. Anschließend wird die Doppelemulsion durch Zugabe einer wässrigen Detergenz-Lösung (Tween 80 in Puffer) zur Einfachemulsion und dreimalige Extrusion durch eine 12 µm Whatman Membran hergestellt.

*E. coli* ist der im Stand der Technik meistgenutzte Wirtsstamm bei der Erstellung von Metagenombanken. Daher wurden bisher bereits einige Studien zur Überwindung der vorgenannten Expressionsproblematik durchgeführt, die sich zumeist mit verbesserten Vektorsystemen beschäftigten. Auch wurden andere Nicht-*E*. *coli*-Wirtsstämme entwickelt, welche einige Probleme in der Faltung und Sekretion adressierten. Trotz alledem bleibt *E. coli* der Wirtsstamm zur effektivsten Metagenombankerstellung. Eine vor kurzem durchgeführte Transkriptomanalyse mit *E. coli*-Stämmen, die Metagenomfosmide tragen, hat allerdings gezeigt, das *E. coli* nur ca. 10 - 20 % aller Promotoren erkennt und in einem Maße abliest, das genügend Protein gebildet wird, um produzierte Enzyme in einem nachgeschalteten Aktivitätstest identifizieren zu können (siehe Lit. 6). Somit stellt die Transkription einen limitierenden Schritt für die Durchmusterung von Metagenombanken dar. Darüber hinaus gibt es im Stand der Technik gerade im Bereich der funktionalen Metagenomik etliche Bemühungen, Hochdurchsatzverfahren für die Durchmusterung von Metagenombanken zum Einsatz zu bringen. Üblicherweise zielt dies jedoch immer auf die Optimierung der Erstellung der Metagenombanken hin; oder aber es wird ein "genetischer Screen" angestrebt. Dabei wird in der Regel eine *E. coli-*Mutante (oder wenige andere Gram-negative Mikroorganismen) in einem essentiellen Gen komplementiert. Der Durchsatz wird dabei im Stand der Technik durch die Transformationseffizienz begrenzt.

Der Erfindung liegt somit die Aufgabe zugrunde, die vorstehend beschriebenen Nachteile des Standes der Technik zu beheben und leistungsfähige bzw. effiziente Ultra-Hochdurchsatz-Durchmusterungssysteme für das Protein-Engineering, insbesondere für die Gelenkte Evolution, und/oder das Metagenomscreening zur Verfügung zu stellen.

Zur Lösung dieser Aufgabe wird nachfolgend eine erfindungsgemäße Technologie offenbart und in den Ansprüchen definiert, welche die zellfreie Expression mit Polymervesikel- bzw. Einfachemulsion-basierten Kompartimenten und der Gelenkten Evolution kombiniert, und welche so bisher nicht im Stand der Technik beschrieben bzw. verfügbar war.

In einer Ausführungsform betrifft die Erfindung ein Verfahren zur Herstellung einer Vielzahl von zellfreien Kompartimenten für die Durchmusterung von (i) Enzymvarianten und/oder (ii) Varianten fluoreszierender Proteine umfassend die Schritte:
a) Bereitstellen einer Vielzahl von Genvarianten,
b) Bereitstellen einer Vielzahl eines *in vitro* Expressionssystems,
c) Bereitstellen, im Falle von (i), einer Vielzahl mindestens eines Substrates für das Enzym, dessen Varianten durchgemustert werden sollen,
d) Bereitstellen zur Bildung von Polymervesikeln geeigneter Bausteine und
e) Bildung von einer Vielzahl von Polymervesikeln aus den Bausteinen als zellfreie Kompartimente unter Einschluss von wenigstens einer Genvariante, bevorzugt unter Einschluss von genau einer Genvariante, des *in vitro*-Expressionssystems und, im Falle von (i), des Substrates, wobei die Polymervesikel Einfachkompartimente sind.

Zur Bildung von Polymervesikeln geeignete Bausteine im Sinne der vorliegenden Erfindung sind Monomere, Multimere (2 - 50 Untereinheiten eines oder mehrerer Monomere), fluoreszierende bzw. nicht-fluoreszierende Copolymere und Polymere oder Mischungen daraus. Besonders bevorzugt sind die in den unten stehenden Tabellen genannten Bausteine.

In einer bevorzugten Ausführungsform betrifft die Erfindung ein Verfahren für die Durchmusterung von Enzymvarianten oder von Proteinvarianten in zellfreien Kompartimenten, umfassend die Schritte:
Durchführen eines Verfahrens wie vorher beschrieben mit den Schritten a) bis e); und
   f) Schaffen von Bedingungen, die die *in vitro* Expression der Genvarianten in den Polymervesikeln bewirken und
   g) Sortieren der Polymervesikel in Abhängigkeit von der Umsetzung des Substrates oder der Generierung eines Fluoreszenzsignals.

Gegenstand der Erfindung ist somit ein neues Kompartiment-basiertes, zellfreies Durchmusterungsverfahren zum (1) Auffinden von Proteinen mit veränderten Eigenschaften (z.B. Stabilität, Aktivität) aus Gelenkten Protein-Evolutionsexperimenten und (2) zum Auffinden neuer Proteine in Metagenomen. Die Proteine können beispielsweise Enzyme sein, Die Kompartimente enthalten im Inneren eine wässrige Phase, die von einer Polymerhülle umgeben ist, die bevorzugt eine Vesikel- oder Stäbchenform aufweisen. Die wässrige Phase der Kompartimente enthält - neben einer Genvariante - ein zellfreies Expressionssystem, welches aus einer *in vitro*-Transkriptions- und Translationsmaschinerie besteht, die die Proteinvariante herstellt. Ferner wird im Kompartiment im Falle durchzumusternder Enzyme ein Substrat von aktiven Enzymvarianten in ein Produkt umgesetzt, das im Kompartiment verbleibt und eine Detektion mit anschließender Isolation erlaubt, z.B. mittels Durchflusszytometrie.

Bevorzugte *in vitro* Transkriptions- und Translationssysteme sind: Kaninchenretikulozytenextrakt, Insektenzellextrakt (z.B. Extrakt von *Spodoptera frugiperda),* Weizenkeimextrakt oder CHO-Zellextrakt als eukaryontische Systeme und *E. coli*-Extrakt als prokaryontisches System.

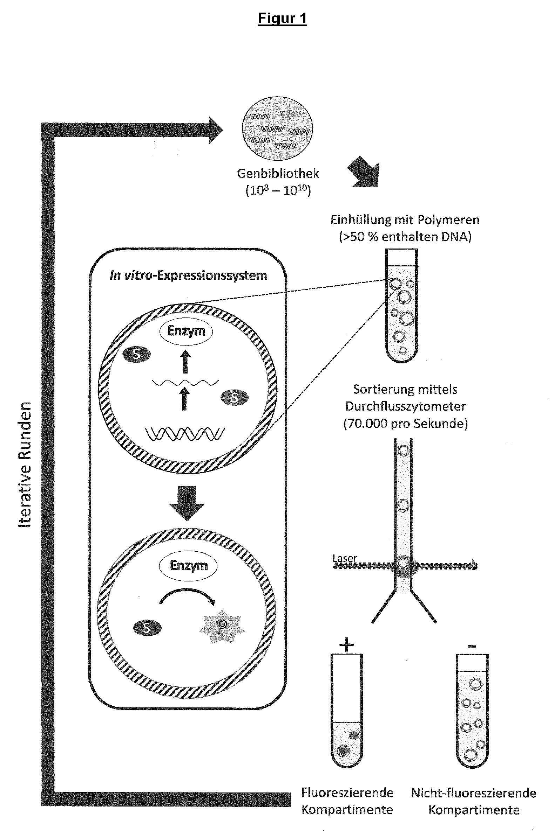

Das erfindungsgemäße Verfahren ermöglicht die deutlich schnellere und effizientere Identifikation von verbesserten Enzymvarianten mit extrem hohen Durchsätzen bei der Gelenkten Enzym-Evolution und Durchmusterung nach neuen Enzymen aus Metagenomen bzw. bei der Gelenkten Protein-Evolution und Durchmusterung nach neuen Proteinen aus Metagenomen. Die Erfindung wird beispielhaft durch die Figur 1 weiter verdeutlicht.
- **Figur 1:**: Beispielhafter Verfahrensweg des erfindungsgemäßen Kompartimentbasierten zellfreien Durchmusterungsverfahren zur schnellen und effizienten Identifikation von verbesserten Proteinvarianten aus Gelenkten Enzym-Evolutionsexperimenten bzw. von neuen Proteinen aus Metagenomen. Im vorliegenden Beispiel erfolgt die Einhüllung mit Polymeren und es entstehen Polymervesikel, die mittels Durchflusszytometrie sortiert werden können. Geeignete Varianten können isoliert und für weitere Gelenkte Evolutionsrunden genutzt werden.

Die Erfindung betrifft somit ein neues zellfreies Durchmusterungsverfahren zum Auffinden von neuen Proteinen oder Proteinen mit veränderten Eigenschaften und die Bereitstellung hierfür geeigneter Mittel. Hierbei zeichnet sich die Erfindung gegenüber dem Stand der Technik dadurch aus, dass für das Expressionssystem ein zellfreies Einfachkompartiment mit Polymerhülle hergestellt und verwendet wird. Solche Polymer-basierten zellfreien Kompartimente sind per se neu, da sie mit einem zellfreien Expressionssystem versehen sind.

Das im Rahmen der Erfindung beschriebene zellfreie System unterscheidet sich hierbei u.a. auch durch die Generierung, Bereitstellung und/oder Verwendung von Polymervesikeln von dem Stand der Technik; bei zellfreien Systemen sind die üblichen Doppelemulsionen (ohne Polymerhülle) der Stand der Technik.

Die vorliegende Erfindung besitzt vielfältige Anwendungsmöglichkeiten, wie insbesondere im Bereich der Veränderung von bereits bekannten Enzymen und/oder Proteinen und dem Auffinden von bisher unbekannten Enzymen und/oder Proteinen. Insbesondere ist es mit der Erfindung möglich, sowohl toxische als auch humane Enzyme und/oder Proteine zu evolvieren.

Bevorzugt zeichnen sich die vorstehend genannten erfindungsgemäßen Verfahren, insbesondere in der bevorzugten Ausführungsform mit den Schritten f) und g), dadurch aus, dass die Polymervesikel durch ein Verfahren gebildet werden, ausgewählt aus der Gruppe bestehend aus Extrusion, Ultraschallbehandlung, Elektroformation, Selbstassemblierung, Protein-vermittelter Assemblierung in wässriger Lösung, Homogenisation, wiederholtes Einfrieren und Auftauen, direkte Dispersion, Ethanol-Methode, Ink Jet-Methode, Filmhydrationsmethode und Polymerisierung an der Phasengrenzfläche von Wasser-in-Öl-Emulsionen.

Weiter bevorzugt zeichnen sich die vorstehend genannten erfindungsgemäßen Verfahren, insbesondere in der bevorzugten Ausführungsform mit den Schritten f) und g), dadurch aus, dass die zur Bildung von Polymervesikeln geeigneten Bausteine Monomere, Multimere, fluoreszierende bzw. nicht-fluoreszierende Di- oder Triblock-Copolymere und Polymere sind. Besonders bevorzugte zur Bildung von Polymervesikeln geeignete Bausteine sind Copolymere, vorzugsweise Di- oder Triblock-Copolymere.

In diesem Zusammenhang bevorzugte Monomere und Copolymere finden sich weiter unten im Text.

Bevorzugt zeichnen sich die vorstehend genannten erfindungsgemäßen Verfahren, insbesondere in der bevorzugten Ausführungsform mit den Schritten f) und g), dadurch aus, dass das Substrat für das Enzym der zur Bildung von Polymervesikeln geeignete Baustein ist oder wobei letzterer unmittelbar oder mittelbar mit einem Umsetzungsprodukt des Substrates für das Enzym reagiert.

Im Rahmen dieser vorstehend genannten besonderen Ausführungsformen sind die Copolymere bevorzugt ausgewählt aus der Gruppe bestehend aus nicht enzymatisch abbaubaren Polymervesikel bildenden Copolymeren, enzymatisch abbaubaren Polymervesikel bildenden Copolymeren und Stimuli-sensitiven Polymervesikel bildenden Copolymeren.

Copolymere oder Heteropolymere sind Polymere, die aus zwei oder mehr verschiedenartigen Monomereinheiten zusammengesetzt sind. Polymervesikel können aus Di- oder Triblock-Copolymeren aufgebaut werden. Eine Auflistung bevorzugter Copolymer-Kombinationen zur Verwendung für die Polymervesikel-Bildung ist in den Tabellen 1 und 2 angegeben.

Die Copolymere, ebenso wie Tenside, bestehen allgemein aus einem hydrophoben ("wasserabweisenden") und einem hydrophilen ("wasserliebenden") Molekülteil; man sagt, sie sind amphiphil ("beides liebend"). Im Gegensatz zu Tensiden können Copolymere aber aus mehreren hundert Untereinheiten bestehen und im Gegensatz zu Tensiden (Micellen) Vesikel mit wässriger innerer Phase ausbilden. Der Aufbau von Polymervesikeln und Tensiden kann durch nichtionische, anionische, kationische und/oder amphotere Gruppen geschehen. Die Block-Copolymer-Kettenlänge kann bei der Synthese verändert werden, um Polymervesikel mit der gleichen Struktur, aber unterschiedlichen Dicken zu ergeben.

Eine terminale Polymer-Gruppe kann bevorzugt in Block-Copolymer-Ketten chemisch modifiziert werden, um veränderte Polymervesikel zu ergeben, die für einige Anwendungen wie Polymervesikel-Immobilisierung von Interesse sein können. Die Bildung von Riesenvesikeln ist möglich, unter Verwendung von Block-Copolymeren und durch einige Methoden wie Elektroformation. Membranproteine oder jede andere Struktur, die den hohlen Kanal-Innenraum bildet, können leicht in die Polymervesikel-Struktur eingebaut werden, um Moleküle und/oder Spezies zu überführen.

Zur Herstellung von Polymervesikeln sind viele Methoden verfügbar. Die wichtigsten Herstellungsmöglichkeiten können in zwei Gruppen eingeteilt werden: Lösemittel-Wechsel-Techniken ("solvent-switching techniques") und Polymer-Rehydration-Techniken ("polymer rehydration techniques"):
- Für Herstellung von Polymervesikeln mittels Lösemittel-Wechsel-Techniken ("solvent-switch techniques") werden Copolymere in organischem Lösemittel gelöst und anschließend hydriert.
- Die Herstellung von Polymervesikeln mittels Polymer-Rehydration-Techniken ("polymer rehydration techniques") basiert auf der Hydrierung von amphiphilen Block-Copolymer Filmen zur Induzierung der Selbst-Zusammenlagerung ("selfassembly") der Block-Copolymere zu Polymervesikeln.

Des Weiteren können Polymervesikel durch Polymerisierung von Monomeren, Multimeren und/oder Copolymeren an der Grenzfläche von Einfachkompartimenten (z.B. Wasser-in-Öl-Einfachemulsionen) hergestellt werden.

Als nicht enzymatisch abbaubare Polymervesikel bildende Copolymere werden im Rahmen der Erfindung verstanden (siehe auch Tabelle 1): Copolymere, die zwar durch chemisch-physikalische Einflüsse jedoch nicht durch enzymatische Aktivität zersetzt werden können.

Als enzymatisch abbaubare Polymervesikel bildende Copolymere werden im Rahmen der Erfindung verstanden (siehe auch Tabelle 1): Copolymere, die sowohl durch chemisch-physikalische Einflüsse als auch durch enzymatische Aktivität zersetzt werden können.

Als stimuli-sensitive Polymervesikel bildende Copolymere werden im Rahmen der Erfindung verstanden (siehe auch Tabelle 2): Copolymere, die durch Anwendung eines oder mehrerer (z.B. gezielter) externer Stimuli (insbesondere pH-, Temperatur-Änderung) reversibel ihre Eigenschaften ändern können.

**Tabelle 1: Beispiele Block-Copolymere (nicht enzymatisch abbaubare und enzymatisch abbaubare)**

| **Block-Copolymere** | **Herstellmethode** | **Wirkstoff / Stimulus für die Freisetzung** |
|---|---|---|
| **Nicht enzymatisch abbaubare Polymervesikel:** | | |
| PEG-PEE | Film Rehydration | |
| PEG-PBD | Film Rehydration | Paclitaxel / Doxorubicin |
| PMOXA-PDMS-PMOXA | Phasen-Inversion | UV-Crosslinking / Calcein |
| PEG-PS | Phasen-Inversion | |
| PAA-PS | Phasen-Inversion | |

| **Enzymatisch abbaubare Polymervesikel:** | | |
|---|---|---|
| PEG-PLA/ phase inversion/ carboxyl fluorescein | Phasen-Inversion | |
| PEG-PCL | Phasen-Inversion | Carboxylfluorescein |
| PEG-PTMC | Phasen-Inversion | |
| PEG-PTMBPEC | Phasen-Inversion | Paclitaxel, Doxorubicin / pH-gesteuerte Hydrolyse |
| Außerdem ist es möglich enzymatisch abbaubare Polymervesikel aus Polypeptiden aufzubauen, die entweder aus Polymer-Peptid Hybriden oder nur aus Peptiden bestehen können. | | |

Die im Rahmen der Erfindung, z.B. hier in Tabelle 1, verwendeten Abkürzungen besitzen folgende Bedeutungen:

### Nicht enzymatisch abbaubare Polymervesikel:

**PEG-PEE:** Poly(ethylenglycol)-b-poly(ethylethylen);
**PEG-PBD:** Poly(ethylenglycol)-b-poly(butadien);
**PEG-BuA:** Poly(ethylenglycol)-butylacrylat;
**PEG-HEMA:** Poly(ethylenglycol)-hydroxyethylmethacrylat;
**PMOXA-PDMS-PMOXA:** Poly(2-methyl-2-oxazolin)-b-poly(dimethylsiloxan)-b-poly(2-methyl-2-oxazolin);
**PEG-PS:** Poly(ethylenglycol)-b-poly(styrol);
**PAA-PS:** Poly(acrylsäure)-b-poly(styrol);
**PVCL:** Poly(N-vinylcaprolactam);
**PVP:** Polyvinylpyrrolidon.

**Enzymatisch abbaubare Polymervesikel:**
**PEG-PLA:** Poly(ethylenglycol)-b-poly(lactid);
**PEG-PCL:** Poly(ethylenglycol)-b-poly(ε-caprolacton);
**PEGPTMC:** Poly(ethylenglycol)-b-poly(trimethylencarbonat);
**PEG-PTMBPEC:** Poly(ethylenglycol)-b-poly(2,4,6-trimethoxybenzylidenpentaerythritolcarbonat).

**Tabelle 2: Beispiele Block-Copolymere (Stimuli-sensitiv)**

| **Stimuli-sensitive Polymervesikel:** | | | |
|---|---|---|---|
| **Block-Copolymere** | **Herstellmethode** | **Enzymatische Abbaubarkeit** | **Wirkstoff / Stimulus für die Freisetzung** |
| PGA-PBD | Basische Lösung | keine | pH-gesteuerte Größenänderung |
| PEG-(PG2MA-IND) | pH 2.0-3.5 | keine | pH-gesteuerte Hydrolyse |
| PEG-P2VP | Phasen-Inversion | keine | Fluorescein / pH-gesteuerte Deformation |
| PLys-PLE | Wasser oder Phasen-Inversion | ja | pH-gesteuerte Deformation |
| PMPC-PDPA | Wasser, pH 2-6 | keine | DNA / Freisetzung bei pH < 6 |
| PAA | Polyelektrolyt | | pH-sensitiv |
| PVIM | Polyelektrolyt | | pH-sensitiv |
| PEG-PS-PDEAMA | Phasen-Inversion | keine | pH-einstellbare Membranpermeabilität |
| PLys-PGA | pH < 4 or pH > 10 | ja | pH (schizophrenisch) |
| PAMPA-PNIPAAm | Wasser, Erhitzen | keine | Temperatur |
| PCEMA-PNIPAAm | Wasser, Erhitzen | keine | Temperatur |
| PEG-PNIPAAm | Wasser, Erhitzen | keine | Doxorubicin / |
| | | | Temperatur |
| PLA-PNIPAAm | Wasser | keine | Temperatur |
| PVCL | | | Temperatur |
| PMEA | | | Temperatur |
| PDEGA | | | Temperatur |
| PTEGA | | | Temperatur |
| PEG-PPS-PEG | Phasen-Inversion | keine | Oxidation |
| PEG-SS-PPS | Film-Rehydration | keine | Calcein / Reduktion |
| PAA-PAzoMA | Phasen-Inversion | keine | Deformation durch UV-Licht |
| PGA-PBD γ-Fe₂O₃ | in Wasser | keine | magnetisches Feld |
| PEG-PI / PEG-P2VP /Fe₃O₄ | magnetisches Feld | keine | magnetisches Feld |

Die im Rahmen der Erfindung, z.B. hier in Tabelle 2 ergänzend zu Tabelle 1, verwendeten Abkürzungen besitzen folgende Bedeutungen:
**Stimuli-sensitive Polymervesikel:**
   **PAA:** Polyacrylsäure;
   **PVIM:** Poly(N-vinylimidazol);
   **PGA-PBD:** Poly(*L*-glutaminsäure)-b-poly(butadien);
   **PEG-(PG2MA-IND):** Poly(ethylenglycol)-b-poly(glycerinmonomethacrylat)-IND;
   **PEG-P2VP:** Poly(ethylenglycol)-b-poly(2-vinylpyridin);
   **PLys-PLE:** Poly(*L*-lysin)-b-poly(leucin);
   **PMPC-PDPA:** Poly(2-(methacryloyloxy) ethylphosphorylcholin)-b-poly(2-(diisopropylamino) ethylmethacrylat);
   **PEG-PS-PDEAMA:** Poly(ethylenglycol)-b-poly(styrol)-b-(poly(2-diethylaminoethylmethacrylat);
   **PLys-PGA:** Poly(*L*-lysin)-b-poly(L-glutaminsäure);
   **PAMPA-PNIPAAm:** Poly(*N*-(3-aminopropyl)-methacrylamidhydrochloride)-b-poly(*N*-isopropylacrylamid);
   **PCEMA-PNIPAAm:** Poly(2-cinnamoylethylmethacrylat)-b-poly(*N-*isopropylacrylamid);
   **PEG-PNIPAAm:** Poly(ethylenglycol)-b-poly(*N*-isopropylacrylamid);
   **PLA-PNIPAAm:** Poly(lactid)-b-poly(*N*-isopropylacrylamid);
   **PEG-PPS-PEG:** Poly(ethylenglycol)-b-poly(propylensulfid)-b-poly(ethylenglycol);
   **PEG-SS-PPS:** Poly(ethylenglycol)-disulfid gebundenes Poly(propylensulfid);
   **PAA-PAzoMA:** Poly(acrylsäure)-b-poly(methacrylat) enthaltend eine Seitenkette von Azobenzol;
   **PEG-PI:** Poly(ethylenglycol)-b-poly(isopren).

Werden Monomere bzw. Multimere als zur Bildung von Polymervesikeln geeignete Bausteine verwendet, werden folgende Monomere bevorzugt eingesetzt (siehe Tabelle 3).

**Tabelle 3: Beispiele Monomere**

| |
|---|
| Styrol und funktionalisierte Styrol-Monomere (wie 4-Benzhydrylstyrol, 2,4-Dimethylstyrol, 2,5-Dimethylstyrol und 4-Vinylbiphenyl) |
| Vinylester (wie Vinylacetat, Vinylbenzoat und Vinyl-4-*tert*-butylbenzoat) |
| Vinyl-Monomere (wie N-Ethyl-2-Vinylcarbazol, 1-Vinylnaphtalen und 2-Vinylnaphtalen) |
| 4-Phenylvinylsulfon und dessen zur Polymerisierung befähigten Derivate |
| Vinyltrimethylsilan und dessen zur Polymerisierung befähigten Derivate |
| 4-Phenylazobenzoylchlorid und dessen zur Polymerisierung befähigten Derivate |
| Acrylat und dessen zur Polymerisierung befähigten Derivate |
| Methacrylat und dessen zur Polymerisierung befähigten Derivate |
| Acrylamid und dessen zur Polymerisierung befähigten Derivate |
| *n*-Butansäure und deren zur Polymerisierung befähigten Derivate |
| Anmerkung: Abhängig vom eingesetzten Monomer bzw. von der Monomer-Kombination kann die Anwesenheit eines zusätzlichen, dem Fachmann bekannten, Polymerisierungsinitiators notwendig sein. |

Für die Ausbildung der Kompartimente zur *in vitro*-Expression in Kompartimenten bestehen verschiedene alternative Möglichkeiten, wie beispielsweise:
- Kombination von Polymervesikeln und Hydrogelen, insbesondere Hydrogele hergestellt durch enzyminitiierte Polymerisierung (Fur-Shell); Hydrogel in Polymervesikeln; Hydrogel an der äußeren Polymervesikeloberfläche;
- Polymervesikel mit vernetzten Gruppen zur Stabilisierung (zum Beispiel PEG-Diacrylat als Copolymerbestandteil zur Polymerisierung);
- Interpenetrierende Netzwerke;
- Polymervesikel können aus unilamellaren oder multilamellaren Membranen aufgebaut werden;

Bevorzugt zeichnen sich die vorstehend genannten erfindungsgemäßen Verfahren, insbesondere in der bevorzugten Ausführungsform mit den Schritten f) und g), auch dadurch aus, dass das Innere der Polymervesikel eine wässrige Lösung ist.

Bevorzugt zeichnen sich die oben beschriebenen erfindungsgemäßen Verfahren auch dadurch aus, dass der Schritt g) mittels eines Verfahrens erfolgt, ausgewählt aus der Gruppe bestehend aus Durchflusszytometrie mit Sortierfunktion, Sortierung mittels mikrofluidischer Chips, Sortierung mittels optischer Mikropinzetten und Sortierung mittels Mikrodissektionssystemen.

In einer weiteren bevorzugten Ausführungsform der oben beschriebenen erfindungsgemäßen Verfahren, insbesondere in der bevorzugten Ausführungsform mit den Schritten f) und g), erfolgt die Isolation der sortierten Genvarianten aus den Polymervesikeln mittels eines Verfahrens, ausgewählt aus der Gruppe bestehend aus Behandlung mit Detergenzien bzw. organischen Lösungsmitteln, enzymatische Behandlung, Behandlung mit mechanischen Kräften, und Einsatz von pH-, Licht-, Temperatur-Stimuli bei Stimuli-sensitiven Polymervesikeln.

Bevorzugte der oben beschriebenen erfindungsgemäßen Verfahren, insbesondere in der bevorzugten Ausführungsform mit den Schritten f) und g), enthalten nach Schritt e) ≥ 50% der gebildeten Polymervesikel wenigstens eine Genvariante.

Bevorzugte der oben beschriebenen erfindungsgemäßen Verfahren, insbesondere in der bevorzugten Ausführungsform mit den Schritten f) und g), enthalten ≥ 90% der mindestens eine Genvariante enthaltenden Polymervesikel ein bis drei Genvarianten, wovon wiederum wenigstens 20% nur genau eine Genvariante enthalten.

Die nachfolgende weitere Beschreibung der Erfindung soll die Kompartiment-basierten, zellfreien Ultra-Hochdurchsatz-Durchmusterungssysteme für das Protein-Engineering noch detaillierter erläutern.

Gegenstand der Erfindung ist, wie oben bereits erwähnt, ein neues Kompartiment-basiertes, zellfreies Durchmusterungsverfahren zum (1) Auffinden von Proteinen mit veränderten Eigenschaften (z.B. Stabilität, Aktivität) aus Gelenkten Protein-Evolutionsexperimenten und (2) zum Auffinden neuer Proteine in Metagenomen. Die Proteine können beispielsweise Enzyme sein. Die Kompartimente enthalten im Inneren eine wässrige Phase, die von einer Polymerhülle oder einer Grenzfläche umgeben ist, die bevorzugt eine Vesikel- oder Stäbchenform aufweisen (z.B. Polymervesikel oder Einfachemulsionssysteme wie Wasser-in-Öl). Die wässrige Phase der Kompartimente enthält - neben einer Genvariante - ein zellfreies Expressionssystem, welches aus einer *in vitro*-Transkriptions- und Translationsmaschinerie besteht, die die Proteinvariante herstellt. Ferner wird im Falle durchzumusternder Enzyme im Kompartiment ein Substrat von aktiven Enzymvarianten in ein Produkt umgesetzt, das im Kompartiment verbleibt und eine Detektion mit anschließender Isolation erlaubt (z.B. mittels Durchflusszytometrie).

Bei der Durchflusszytometrie wird ein fluoreszierendes Produkt detektiert mit anschließender elektrostatischer oder mechanischer Sortierung/Isolierung der Kompartimente, die bei einer möglichen Ausführungsform in Gegenwart enzymatischer Aktivität gebildet werden können. Polymer-basierte Kompartimente, deren Polymerhülle durch die Enzymvariante oder gekoppelte Reaktionen gebildet werden, können alternativ z.B. durch Zugabe eines Detergens isoliert werden. Das Verfahren ermöglicht die deutlich schnellere und effizientere Identifikation von verbesserten Enzymvarianten mit extrem hohen Durchsätzen bei der Gelenkten Protein-Evolution und Durchmusterung nach neuen Proteinen aus Metagenomen.

Die Erfindung umfasst ein neues Kompartiment-basiertes zellfreies Durchmusterungsverfahren, das die deutlich schnellere und effizientere Identifikation von verbesserten Proteinvarianten aus Gelenkten Protein-Evolutionsexperimenten bzw. von neuen Proteinen aus Metagenomen ermöglicht. Ein Beispiel für einen möglichen Verfahrensweg ist in Fig. 1 gezeigt. Dabei sind die Art der Kompartimente und die Nutzung der zellfreien Expression besonders hervorzuheben.

Um mit Hilfe des vorgestellten Verfahrens bei der Gelenkten Protein-Evolution eine verbesserte Proteinvariante bzw. aus einem Metagenom ein neues Protein zu identifizieren, werden für das Protein kodierende Genvarianten in Kompartimenten eingekapselt. Dabei wird angestrebt, dass sich nur eine einzige Genvariante innerhalb eines Kompartiments befindet.

Einfachemulsionskompartimente können z.B. über die robuste und einfache Methode der Extrusion generiert werden. Bei Polymerkompartimenten besteht die Hülle aus Di-/Triblock Copolymeren unterschiedlicher Kettenlängen, wie beispielsweise Polyethylenglykol-b-Polypropylenglykol, PMOXA-PDMS-PMOXA oder Polyethylenglykol-b-Polycaprolacton. Die Hülle der Polymerkompartimente kann dabei über die Zusammensetzung der einzelnen Blöcke frei gewählt und auf die jeweilige Anforderung zugeschnitten werden. Zur Herstellung der Polymerkompartimente wird das gewählte Copolymer in eine wässrige Lösung gegeben und zur Selbst-Assemblierung z.B. mit Ultraschall behandelt und gerührt oder alternativ mittels "Film-Rehydration" hergestellt.

Eine weitere Option ist, dass die Polymerhülle selber durch Polymerisierung herstellt gestellt wird, welche auf Substratumsatz durch die aktive Enzymvariante basiert, z.B. durch eine H₂O₂ initiierte Polymerisation.

Die wässrige Phase der Kompartimente enthält - neben einer Genvariante - ein zellfreies Expressionssystem, welches aus einer *in vitro*-Transkriptions- und Translationsmaschinerie (z.B. Insektenzell- oder Weizenkeimextrakte) besteht, die die Proteinvariante herstellt. Ferner wird bei einer Ausführungsform im Kompartiment ein Substrat von aktiven Enzym-varianten in ein Produkt umgesetzt, das im Kompartiment verbleibt und eine Detektion mit anschließender Isolation erlaubt, z.B. mittels Durchflusszytometrie. Die Nutzung von zellfreien Expressionssystemen wie Insektenzell- oder Weizenkeimextrakten ermöglicht erstmals eukaryontische Gene und toxische Proteine den Methoden der Gelenkten Evolution zuzuführen und die Umgehung von Organismen (inkl. Klonierung und Expression), ermöglicht eine massive Beschleunigung der Gelenkten Evolution mit zahlreichen Wettbewerbsvorteilen für Firmen, die diese Methoden nutzen können.

Die anschließende Hochdurchsatzdurchmusterung der Varianten kann z.B. mittels eines Durchflusszytometers erfolgen, welches die Durchmusterung von 10⁷ - 10⁸ Varianten innerhalb einer Stunde ermöglicht. Die Isolierung der aktiven Varianten erfolgt dabei durch das Durchflusszytometer, welches aktivitätsverbesserte, aktivitätsreduzierte und inaktive Varianten voneinander trennt. Durch die Möglichkeit solch eine hohe Anzahl an Varianten durchzumustern, können neue Gelenkte Evolutions-Strategien mit hohen Mutagenesefrequenzen (< 1 % aktive Varianten) ausgeführt werden. Zudem ist es im Falle von Metagenombanken wahrscheinlicher, wirklich neue Enzyme aufzufinden (Hitrate von Monooxygenasen: eine in einer Million Klone), wenn möglichst viele Varianten durchgemustert werden.

Eine Alternative zur Fluoreszenz-basierten Sortierung mittels Durchflusszytometer ist die Isolierung von Kompartimenten mit Polymerhülle. Dabei ist die Bildung der Polymerhülle des Einfachkompartiments (wie z.B. Wasser-in-Öl-Einfachemulsion) entweder direkt oder indirekt an die Gegenwart katalytischer Aktivität geknüpft, die bevorzugt enzymatischer und/oder biohybridkatalysatorischer und/oder gekoppelt chemo-enzymatischer Natur ist. Wird innerhalb des Kompartiments eine aktive Variante exprimiert, wird der Polymerisationsprozess in Gang gesetzt, sodass sich die Polymerhülle (z.B. an der Grenzfläche von Wasser-in-Öl-Einfachemulsion) ausbildet. Aktive Varianten können z.B. durch Zugabe eines Detergens isoliert werden, da dadurch Kompartimente ohne Polymerhülle im Gegensatz zu Kompartimenten ohne Polymerhülle brechen. Nach der Isolation der verbesserten Varianten bzw. der neuen Enzyme, können weitere Analysen durchgeführt und die gefundenen Varianten charakterisiert werden. Im Fall des iterativen Prozesses der Gelenkten Evolution kann die nächste Evolutionsrunde angeschlossen werden.

Katalytische Aktivität enzymatischer Natur im Sinne der im vorigen Abschnitt beschriebenen Verfahrensform geht von Protein-basierten Enzymen aus. Katalytische Aktivität biohybridkatalysatorischer Natur im Sinne der im vorigen Abschnitt beschriebenen Verfahrensform geht von Biohybridkatalysatoren aus, die aus einem nicht-katalytischen Proteingerüst und einem daran gebundenen chemischen Katalysator, wie z. B. organometallische Verbindungen oder Organokatalysatoren, bestehen. Katalytische Aktivität gekoppelt chemo-enzymatischer Natur im Sinne der im vorigen Abschnitt beschriebenen Verfahrensform geht von einer Kombination von Katalyse mittels chemischer Katalysatoren mit Katalyse mittels Enzym-basierten Katalysatoren aus. Bei der katalytischen Aktivität gekoppelt chemo-enzymatischer Natur entsteht ein Umsetzungsprodukt durch die zuerst stattfindende Reaktion, welche durch einen oder mehrere der anwesenden Katalysatoren katalysiert wird. Dieses entstehende Produkt dient direkt als Edukt für die anschließend stattfindende Reaktion, welche durch z.B. einen anderen der anwesenden Katalysatoren katalysiert wird etc..

Neben dem oben beschriebenen erfindungsgemäßen Verfahren betrifft die Erfindung auch die Verwendung einer Vielzahl von Polymervesikeln, wie diese in einem der oben offenbarten erfindungsgemäßen Verfahren, insbesondere in der bevorzugten Ausführungsform mit den Schritten f) und g), definiert sind, als Einfachkompartimente in einem Verfahren zur Herstellung einer Vielzahl von zellfreien Kompartimenten für die Durchmusterung von (i) Enzymvarianten und/oder (ii) Varianten fluoreszierender Proteine oder in einem Verfahren für die Durchmusterung von (i) Enzymvarianten und/oder (ii) Varianten fluoreszierender Proteine. Die im Zusammenhang mit dem erfindungsgemäßen Verfahren dargelegte Offenbarung gilt gleichermaßen, sowohl für einzelne Merkmale als auch für deren Kombinationen, für die erfindungsgemäße Verwendung.

Die Erfindung betrifft darüber hinaus als Gegenstand auch eine Vielzahl von Polymervesikeln hergestellt oder herstellbar nach einem der oben offenbarten erfindungsgemäßen Verfahren, insbesondere in der bevorzugten Ausführungsform mit den Schritten f) und g). Die im Zusammenhang mit dem erfindungsgemäßen Verfahren dargelegte Offenbarung gilt gleichermaßen, sowohl für einzelne Merkmale als auch für deren Kombinationen, für die erfindungsgemäße Vielzahl von Polymervesikeln.

Ferner betrifft die Erfindung als Gegenstand auch eine Fraktion von Polymervesikeln umfassend ein Gen für eine aktive Variante des durchzumusternden (i) Enzyms und/oder (ii) fluoreszierenden Proteins, gewinnbar oder gewonnen durch ein bevorzugtes Verfahren in der Ausführungsform mit den Schritten f) und g), oder durch ein mit einem oder mehreren wie oben beschriebenen Merkmalen weitergebildetes Verfahren der Ausführungsform mit den Schritten f) und g). Die im Zusammenhang mit dem erfindungsgemäßen Verfahren dargelegte Offenbarung gilt gleichermaßen, sowohl für einzelne Merkmale als auch für deren Kombinationen, für die erfindungsgemäße Fraktion von Polymervesikeln.

Die technischen Anwendungsgebiete der vorliegenden Erfindung sind sehr vielfältig und umfassen jeglichen Bereich, wo die Optimierung von Enzymen oder Proteinen bzw. das Auffinden neuer Enzyme oder neuer Proteine von Bedeutung ist, also industrielle und universitäre Forschung und Entwicklung. Auf der industriellen Seite sind vornehmlich die Waschmittel-, Kosmetik-, Textil-, Pharma- und Lebensmittel- und Futtermittelindustrie als Beispiele zu nennen. Im universitären Bereich eignet sich die vorliegende erfindungsgemäße Technologie beispielsweise sehr gut zur Untersuchung von Struktur-Funktionszusammenhängen und das Auffinden strukturell neuer Enzyme.

Ein herausragender Vorteil der Erfindung besteht darin, dass viele Enzyme, die bisher mangels Expressionsmöglichkeit nicht evolviert werden konnten (z.B. humane oder toxische Proteine), dank der erfindungsgemäßen Technologie - also wie hier anhand der erfindungsgemäßen Verfahren, der erfindungsgemäßen Verwendung, der erfindungsgemäßen Vielzahl von Polymervesikeln oder der erfindungsgemäßen Fraktion von Polymervesikeln beschrieben - dem Protein-Engineering zugänglich gemacht werden und darüber hinaus verbesserte Varianten bzw. neue Enzyme oder Proteine (aus Metagenomen) schneller und effizienter aufgefunden werden können. Damit hat das erfindungsgemäße Verfahren enormes Potential, in die Entwicklung von industriellen Enzymprozessen integriert zu werden und medizinisch bedeutsame Proteine erstmals zu evolvieren.

Die im erfindungsgemäßen Verfahren genutzten Kompartimentsysteme besitzen im Vergleich zu Doppelemulsionen des Standes der Technik mehrere Vorteile. So ist die

Herstellung der oben beschriebenen erfindungsgemäßen Kompartimentsysteme deutlich einfacher und sie weisen im Vergleich zu bekannten Doppelemulsionen eine erhöhte Stabilität auf und mehrere Kompartimente wie in Doppelemulsionen des Standes der Technik werden erfindungsgemäß nicht eingeschlossen (siehe bspw. Figur 1).

Durch Anwendung des erfindungsgemäßen zellfreien Expressionssystems wird die ansonsten erforderliche Nutzung von Organismen zur Expression vermieden. Somit können sonst notwendige Wachstumszeiten reduziert werden, was den Gelenkten Evolutionsprozess massiv beschleunigt. Bei Nutzung des erfindungsgemäßen Verfahrens reduziert sich im Erfolgsfall der Zeitbedarf pro Gelenkter Evolutionsrunde auf einen Tag, was inklusive der folgenden Durchmusterung der besten Varianten die Realisierung einer kompletten Evolutionskampagne mit sechs iterativen Gelenkten Evolutionsrunden in zwei Wochen ermöglicht. Eine hohe Zahl von Evolutionsrunden ist nötig, um signifikant verbesserte Enzyme bzw. Proteine herzustellen. Für die Gelenkte Evolution in Mikroorganismen gemäß Stand der Technik wird im Vergleich zur erfindungsgemäßen zellfreien Gelenkten Evolution etwa zehnmal so viel Zeit benötigt.

Darüber hinaus bestehen erfindungsgemäß keine Limitierungen aufgrund von Transformationseffizienzen (z.B. wie bei industriell bedeutsamen *Bacillus-*Produktionsstämmen), da erfindungsgemäß kein Transformationsschritt benötigt wird und ohne die Nutzung von Organismen können aufgrund der vorliegenden Erfindung erstmalig auch toxische und humane Proteine effizient evolviert und in Metagenomen aufgefunden werden. Im Falle humaner und toxischer Proteine, die nur in zellfreien Expressionssystemen mit der erfindungsgemäßen entwickelten Technologie und den Methoden der Gelenkten Evolution maßgeschneidert werden können, gibt es im Stand der Technik für deren Verbesserung bisher keine effizienten Alternativen zur erfindungsgemäßen Technologie.

Die nachfolgenden Beispiele sollen die Erfindung weiter erläutern, ohne diese jedoch in ihrem Umfang zu beschränken.

### Beispiele

### In vitro-Expression von Zellulasevarianten in Polymervesikeln

### Beispiel 1:

### Herstellung lyophilisierter Polymervesikel

Polymervesikel dienen als Kompartimente, in denen die *in vitro* Expression stattfindet.

10 mg Triblock-Copolymer Poly(2-methyloxazoline₁₄-b-dimethylsiloxane₆₅-b-2-methyloxazoline₁₄) (PMOXA-PDMS-PMOXA) (Polymer Source Inc.) wurde mit 1 ml Milli-Q Wasser versetzt und über Nacht in einem Glasgefäß mit einem Magnetrührer (Cimarec I Poly 15, Thermo Scientific) bei 500 rpm ("Umdrehungen pro Minute") gerührt. Die trübe Polymerdispersion wurde zweimal auf Eis sonifiziert (Ultraschallbehandlung 3 x 30 s mit jeweils 30 s Pausenintervallen zur Kühlung auf Eis; Amplitude: 30 (VCX 130, Sonics & Materials Inc.)). Zwischen beiden Ultraschallbehandlungen wurde die Polymerdispersion für eine Stunde mit einem Magnetrührer bei 500 rpm gerührt.

Daraufhin wurden 10 aufeinanderfolgende Gefrier-Auftau-Zyklen ("freeze-thaw cycles"), jeweils bestehend aus 3 min Inkubation in flüssigem Stickstoff, gefolgt von 3 min Inkubation bei 60°C im Wasserbad durchgeführt. Anschließend wurden Aliquote mit je 40 µl Polymerdispersion hergestellt und mit flüssigem Stickstoff eingefroren. Das Wasser wurde durch Lyophilisation (Alpha 1-2 LD plus, Christ) über Nacht entfernt und die leeren Polymervesikel anschließend bei -20 °C gelagert.

### Beispiel 2:

### Herstellung einer DNA-Bibliothek basierend auf dem Zellulasegen celA2

CelA2 Genbibliotheken wurden mittels fehleranfälliger Polymerasekettenreaktion (epPCR) hergestellt und über die PLICing-Methode (siehe Lit. 7) kloniert. Als DNA-Templat diente das Plasmid pET22b(+)-CelA2 His288Phe, welches mit folgenden Primern CelA2_for: atgagcgaagaaTGGCAGACCTATGAAAAAG und CelA2_rev: ttattattcggcCACGGTATTCAGGCTATAAAC unter nachstehenden Bedingungen amplifiziert wurde (klein geschriebene Buchstaben deuten auf Phosphothioat-Modifikation des DNA-Rückgrats hin). Die Polymerasekettenreaktionen, bestehend aus folgenden Komponenten (Stock Konzentration in Klammern) 1 µl *Taq* polymerase, 2 µl dNTPs (je 0.2 mM dATP, dGTP, dTTP, dCTP), 1 µl DNA Templat (30 ng/µl), 1 µl MnCl₂ (10 mM), 2 µl Ce-IA2_for Primer (0.4 pmol), 2 µl CelA2_rev Primer (0.4 pmol), 5 µl 10x PCR-Puffer und 36 µl ddH₂O wurde mit folgenden Bedingungen durchgeführt: 5 min Denaturierung bei 94°C; 30 Zyklen 30 s 94°C, 30 s 60°C, 2 min 68°C; abschließende Elongation 5 min bei 68°C.

Zur Amplifikation des Rückgrats diente das DNA-Templat pIX3.0RMT7 mit den Primern pIX_for: gccgaataataaGAATTCGAGCTCGTCG und pIX_rev: ttcttcgctcatGCTAGCACCC wurde (klein geschriebene Buchstaben deuten auf Phosphothioat-Modifikation des DNA-Rückgrats hin). Die Polymerasekettenreaktionen, bestehend aus folgenden Komponenten (Stock Konzentration in Klammern) 0,5 µl PfuS polymerase, 2 µl dNTPs (je 0.2 mM dATP, dGTP, dTTP, dCTP), 1 µl DNA Templat (30 ng/µl), 2 µl CelA2_for Primer (0.4 pmol), 2 µl CelA2_revPrimer (0.4 pmol), 5 µl 10x PCR-Puffer und 36,5 µl ddH₂O wurde mit folgenden Bedingungen durchgeführt: 3 min Denaturierung bei 98°C; 30 Zyklen 20 s 98°C, 20 s 60°C, 2,5 min 72°C; abschließende Elongation 5 min bei 72°C. Im Anschluss wurde die mittels epPCR hergestellte Zellulasebibliothek mit der PLICing Methode kloniert (siehe Lit. 7).

### Beispiel 3:

### Enkapsulierung des Reaktionsansatzes zur zellfreien Enzymexpression in Polymervesikeln

Der Vektor pIX3.0RMTT7 wurde zur *in vitro*-Proteinexpression verwendet und beinhaltet die Zellulasevariante CelA2 His288Phe als Positivkontrolle. Als Negativkontrolle dient der Vektor pIX3.0RMT7/CelA2Glu580Gln mit der für das Substrat Resorufin-β-D-cellobiose inaktive Variante Glu580GIn. Zur Validierung der Methode wurde eine Zellulasebibliothek in den Vektor pIX3.0RMT7 kloniert. Auf dem Vektor befindet sich außerdem unter Anderem eine β-Laktamase (Ampicillinresistenz), ein T7 Promotor, eine Ribosomenbindestelle und ein T7 Terminator. Die Isolierung des Vektors erfolgte aus dem Stamm *E. coli* Dh5α mittels eines Kits zur Plasmidaufreinigung (NucleoSpin® Plasmid, Macherey-Nagel).

Die Komponenten des Reaktionsansatzes zur *in vitro*-Expression (EasyXpress *E. coli* Kit, biotechrabbit GmbH) wurden auf Eis aufgetaut und wie folgt zu einer Reaktion mit dem Gesamtvolumen von 11,5 µl pipettiert: 4 µl Puffer, 1 µl DNA [5 ng], 1 µl Resorufin-β-D-cellobiose [2 mM] (Marker Gene Technologies Inc.), 0,4 µl Chaperon-Mix [1:5 verdünnt in H₂O] (Art. Nr. P7211-02, RiNA GmbH, Berlin), 3,5 µl Zellextrakt. Die Gesamtreaktion wurde zu den lyophilisierten Polymervesikeln gegeben und für 30 s gevortext. Es folgt eine Inkubation für 30 min auf Eis, um die getrockneten Polymervesikel zu rekonstruieren und mit den für die zellfreie Expression benötigten Komponenten zu beladen. Anschließend wurde eine Protease aus *Bacillus licheniformis* (Sigma P4860) 1:1000 auf 2.4 mU/g mit 10 mM HEPES [pH 7,4] verdünnt und 1 µl zu dem Reaktionsansatz, um eine Proteinsynthese außerhalb der Polymervesikel zu verhindern. Daraufhin wurde die Proteinexpression für 4 h in einem PCR-Cycler (MasterCycler Personal, Eppendorf) durchgeführt.

### Beispiel 4:

### Durchflusszytometriesortierung

Die Durchflusszytometriesortierung der *in vitro* exprimierten und in Polymervesikeln enkapsulierten CelA2-Bibliothek inklusive Positiv- und Negativkontrolle wurde mittels Durchflusszytometer mit Sortierfunktion (BD Influx Cell Sorter, BD Biosciences) durchgeführt. Nach der zellfreien Proteinsynthese *in vitro* in Polymervesikeln wurde der Reaktionsansatz mit 200 µl PBS versehen und filtriert (CellTrics 50 µm, Partec). Die Durchflusszytometrie-Sortierung erfolgte mit PBS pH 7,4 (ThermoFisher Scientific) als Trägerflüssigkeit und einer 100 µm Düse ("nozzle"). Das Enzymprodukt Resorufin wurde mittels eines Lasers (Cobolt Jive, Cobolt AB) bei 561 nm (75 Watt) angeregt und dessen Emission bei mit einem Breitbanddetektor bei 585+/-29 nm gemessen. Geeignete Polymervesikel, die durch Hydrolyse des Substrats Resorufin-β-D-Zellobiose in Resorufin ein höheres Fluoreszenzsignal als die Negativkontrolle zeigten, wurden sortiert.

### Beispiel 5:

### DNA-Rückgewinnung aus Polymervesikeln

Zur DNA-Rückgewinnung aus Polymervesikeln nach Durchflusszytometriesortierung, wurden entsprechende Polymervesikel für 5 min auf 80°C erhitzt und zuvor mit 1 µl Triton X-100 (Sigma-Aldrich)/ 1 ml sortierte Polymervesikel versehen. Anschließend erfolgte eine Aufreinigung der DNA über das NucleoSpin Gel und PCR Clean-up Kit (Macherey-Nagel) und eine Transformation in kompetente *E. coli* DH5α Zellen.

### Literaturliste

1 Tee, K. L. and Schwaneberg, U., Combinatorial Chemistry & High Throughput Screening 10 (3), 197 (2007).
2 Leemhuis, H., Kelly, R. M., and Dijkhuizen, L., IUBMB life 61 (3), 222 (2009).
3 Baret, J.-C. et al., Lab on a chip 9 (13), 1850 (2009);
4 Mastrobattista, E. et al., Chemistry & Biology 12 (12), 1291 (2005).
5 Miller, O. J. et al., Nature Methods 3 (7), 561 (2006)
6 Liebl, W. et al., Applied Microbiology and Biotechnology 98 (19), 8099 (2014).
7 Blanusa, M., Schenk, A., Sadeghi, H., Marienhagen, J., Schwaneberg, U., Analytical Biochemistry. 406 (2), 141 (2010).

## Patentansprüche

1. Verfahren zur Herstellung einer Vielzahl von zellfreien Kompartimenten für die Durchmusterung von (i) Enzymvarianten und/oder (ii) Varianten fluoreszierender Proteine umfassend die Schritte:
a) Bereitstellen einer Vielzahl von Genvarianten,
b) Bereitstellen einer Vielzahl eines *in vitro* Expressionssystems,
c) Bereitstellen, im Falle von (i), einer Vielzahl mindestens eines Substrates für das Enzym, dessen Varianten durchgemustert werden sollen,
d) Bereitstellen zur Bildung von Polymervesikeln geeigneter Bausteine und
e) Bildung von einer Vielzahl von Polymervesikeln aus den Bausteinen als zellfreie Kompartimente unter Einschluss von wenigstens einer Genvariante, bevorzugt unter Einschluss von genau einer Genvariante, des *in vitro* Expressionssystems und im Falle von (i), des Substrates, wobei die Polymervesikel Einfachkompartimente sind.

2. Verfahren für die Durchmusterung von Enzymvarianten oder von Proteinvarianten in zellfreien Kompartimenten, umfassend die Schritte:
Durchführen eines Verfahrens nach Anspruch 1 mit den Schritten a) bis e); und
f) Schaffen von Bedingungen, die die *in vitro* Expression der Genvarianten in den Polymervesikeln bewirken und
g) Sortieren der Polymervesikel in Abhängigkeit von der Umsetzung des Substrates oder der Generierung eines Fluoreszenzsignals.

3. Verfahren nach Anspruch 1 oder 2, wobei die Polymervesikel durch ein Verfahren gebildet werden, ausgewählt aus der Gruppe bestehend aus Extrusion, Ultraschallbehandlung, Elektroformation, Selbstassemblierung, Protein-vermittelter Assemblierung in wässriger Lösung, Homogenisation, wiederholtes Einfrieren und Auftauen, direkte Dispersion, Ethanol-Methode, Ink Jet-Methode, Filmhydrationsmethode und Polymerisierung an der Phasengrenzfläche von Wasser-in-Öl-Emulsionen.

4. Verfahren nach einem der vorangehenden Ansprüche, wobei die zur Bildung von Polymervesikeln geeigneten Bausteine Copolymere, vorzugsweise Di- oder Triblock-Copolymere, sind.

5. Verfahren nach einem der vorangehenden Ansprüche, wobei das Substrat für das Enzym der zur Bildung von Polymervesikeln geeignete Baustein ist oder wobei letzterer unmittelbar oder mittelbar mit einem Umsetzungsprodukt des Substrates für das Enzym reagiert.

6. Verfahren nach Anspruch 4 oder 5, wobei die Copolymere ausgewählt sind aus der Gruppe bestehend aus nicht enzymatisch abbaubaren Polymervesikel bildenden Copolymeren, enzymatisch abbaubaren Polymervesikel bildenden Copolymeren und stimuli-sensitiven Polymervesikel bildenden Copolymeren.

7. Verfahren nach einem der vorangehenden Ansprüche, wobei das Innere der Polymervesikel eine wässrige Lösung ist.

8. Verfahren nach einem der Ansprüche 2 - 7, wobei der Schritt g) mittels eines Verfahrens erfolgt, ausgewählt aus der Gruppe bestehend aus Durchflusszytometrie mit Sortierfunktion, Sortierung mittels mikrofluidischer Chips, Sortierung mittels optischer Mikropinzetten und Sortierung mittels Mikrodissektionssystemen.

9. Verfahren nach einem der vorangehenden Ansprüche, wobei nach Schritt e) ≥ 50% der gebildeten Polymervesikel wenigstens eine Genvariante enthalten.

10. Verfahren nach einem der vorangehenden Ansprüche, wobei ≥ 90% der mindestens eine Genvariante enthaltenden Polymervesikel eine bis drei Genvarianten enthalten, wovon wiederum wenigstens 20% nur genau eine Genvariante enthalten.

11. Verwendung einer Vielzahl von Polymervesikeln wie in einem der vorangehenden Ansprüche definiert als Einfachkompartimente in einem Verfahren zur Herstellung einer Vielzahl von zellfreien Kompartimenten für die Durchmusterung von (i) Enzymvarianten und/oder (ii) Varianten fluoreszierender Proteine oder in einem Verfahren für die Durchmusterung von (i) Enzymvarianten und/oder (ii) Varianten fluoreszierender Proteine.

12. Vielzahl von Polymervesikeln hergestellt oder herstellbar nach einem Verfahren nach einem der Ansprüche 1 - 10.

13. Fraktion von Polymervesikeln umfassend ein Gen für eine aktive Variante des durchzumusternden (i) Enzyms und/oder (ii) fluoreszierenden Proteins, gewinnbar oder gewonnen durch ein Verfahren nach Anspruch 2 oder ein Verfahren nach einem der Ansprüche 3 - 10, jeweils auf Anspruch 2 rückbezogen.
